# EUROPEAN PATENT SPECIFICATION

(11) **EP 2 334 263 B1**
(45) Date of publication and mention of the grant of the patent: **07.01.2015**
(21) Application number: 08812809.5
(22) Date of filing: 30.09.2008
(51) Int. Cl.: A61F 2/30, A61F 2/42

(54) **ANKLE PROSTHESIS AND A TIBIAL COMPONENT THEREFOR**
FUSSGELENKPROTHESE UND TIBIAKOMPONENTE DAFÜR
PROTHÈSE DE CHEVILLE ET COMPOSANT TIBIAL DE CELLE-CI

(43) Date of publication of application: 22.06.2011
(73) Proprietor: Wright Medical Technology, Inc., Memphis, TN 38117 (US)
(72) Inventor: VAN STRATEN, Niels, NL-1011 PG Amsterdam (NL); DOETS, Hendrik Cornelis, NL-1081 HD Amsterdam (NL); VAN STRATEN, Bart-Jan, NL-2585 DD Den Haag (NL)
(74) Representative: Moore, Michael Richard
(86) International application number: PCT/NL2008/050627
(87) International publication number: WO 2010/039026

(56) References cited:
- WO-A-2006/136940
- US-A- 3 886 599
- US-A- 4 069 518
- US-A1- 2005 049 711
- US-A1- 2008 109 081

## Description

The invention relates to a tibial component for use in an ankle prosthesis, wherein said tibial component comprises a base, wherein at an upper surface thereof a fixation fin protrudes generally upwardly from the base.

The invention further relates to an ankle prosthesis, comprising a tibial component, a talar component and a bearing component for placement between said tibial and said talar component.

A disabled ankle joint can lead to significant functional disturbances and to a disturbed gait. This causes additional stress on the knee and hip joint which in turn could cause damage in these joints. Fixed-bearing implants have shown disappointing results and are known to have a high rate of early failures. To help counter the problems with total ankle replacement, Buechel and Pappas developed the B-P Mobile Bearing Total Ankle System. Such a system consists of a tibial component, a talar component, and a bearing component for placement between said tibial and said talar component.

Tibial components for these ankle prostheses are known from the prior art. US 2008/0109081 A1 discloses such a tibial component with a base and an upwards from the base extending rear plate for fixture to a backside of the tibia. One or more spike-like projections extending sideways from the rear plate fix the rear plate to the tibia by extension through a backside of the bone of the tibia. In US 2005/0049711 A1 in name of Robert J. Ball, a tibial component for an ankle prosthesis is described. The tibial component comprises a base. On its upper surface, a bone fixation portion is provided. The bone fixation portion comprises a rectangular portion that is fixed to the base, and a generally cylindrically shaped portion at the upper end of the rectangular portion.

Although the ankle joint has three sets of articulating surfaces, the tibia-talar joint bears the majority of the body weight and the forces on the ankle. A problem of known tibial components is that due to wear, the connection between the tibial component and the tibia is not sufficient. For a large part, this is due to the huge amount of stress that tibial components are subjected to. Due to these stresses, the bone formed around the bone fixation portion of the tibial component may show signs of wear. Eventually, this may lead to a tibial component that is not tightened securely in place and thus has some space for movement in the tibia. This may cause instabilities in the tibial component and in the ankle prosthesis as a whole, and may lead to further wear of the tibia and the tibial component. Also, this may lead to additional stress on the knee and hip joint which in turn could cause damage in these joints.

It is a further disadvantage of the known tibial component that when the tibial component is worn out, replacement of the tibial component is difficult. This is due to the fact that the bone around the bone fixation portion of the tibial component may have been partly worn away. Due to the fact that part of the tibia was already removed for placing the tibial component, a newly placed tibial component may experience problems with securing the tibial component in the tibia. A tight and secure fit may not always be possible, leading to a situation that is far from optimal for a patient.

It is an object of the present invention to provide a tibial component wherein at least one of the aforementioned disadvantages have been removed or at least alleviated. It is a further object of the present invention to provide a tibial component with improved stability.

Thereto, the invention provides a tibial component for use in an ankle prosthesis, wherein said tibial component comprises a base having an upper surface, a fixation fin protruding generally upwardly from the upper surface of the base and extending in an anterior-posterior direction along the base, and at least one stabilisation element provided on the fixation fin for stabilisation of the tibial component when placed in the tibia, said at least one stabilisation element extending sideways away from the fixation fin and at an oblique angle relative to the upward direction of the fixation fin. The stabilisation element on the fixation fin provides further stabilisation of the fin in the tibia. The fin is less able to move in the tibia, and thus scraping of bone that is present around the bone fixation portion is prevented. All in all, the wear of the tibia and the tibial component is decreased, leading to a longer lifetime of the ankle prosthesis as a whole.

In an embodiment of the invention, the tibial component is configured as a replacement tibial component for replacing a worn out tibial component in an earlier placed ankle prosthesis. The worn out tibial component may be a similar tibial component as the tibial component of the present invention. It is also possible that the tibial component of the present invention is used to replace a tibial component that is different from the one of the present invention. For instance, the tibial component to be replaced may lack a stabilisation element. Placement of a tibial component lacking a stabilisation element is relatively easy and requires minimal bone resection. When worn out, replacement of such a tibial component with a tibial component according to the present invention is relatively easy and requires minimal bone resection. However, due to the stabilisation element, the tibial component according to the present invention is securely tightened in the tibia, and therefore enough stabilisation is provided.

In an embodiment of the invention, said at least one stabilisation element extends with a distance, wherein said distance is measured relative to a width of said base, said distance being at least 10% of said width. The size of the stabilisation element is important in providing stabilisation to the tibial component. It has turned out that by extending the stabilisation element with a distance that is equal to 10% of the total width of the base of the tibial component, provides a relatively high stabilisation of the tibial component in the tibia.

Said stabilisation element extends at an oblique angle from said fixation fin. By using an oblique angle, forces on the tibial component are transferred from the fixation fin via the stabilisation elements to the bone that is present around the stabilisation element. Thus, the forces are transferred to a larger area, leading to less stress in the tibial component and in the bone around the tibial component. This reduces the wear of the bone and the tibial component. Moreover, the tibial component according to the present invention is relatively better stabilised.

In an embodiment of the present invention, said angle (•) is between 95° and 155°. The angle is expressed as the smallest angle between the centre line of the fixation fin and the centre line of the stabilisation element. It has turned out that this angle provides a relatively high stabilisation of the tibial component in the tibia. Moreover, it has turned out that this angle provides relatively high resistance against wear of the tibial component and the tibia.

The base of the tibial component may be substantially trapezoidal. It is relatively easy to fit a trapezoidally shaped tibial component in a tibia. The trapezoidal shape offers the advantage that less bone resection is needed for placing the tibial component. The back side or anterior side of the base of the tibial component may have a larger width than the front side or posterior side of the base of the tibial component.

In an embodiment, the trapezoid shape of the base has an anterior-posterior ratio in between 1 and 2, preferably of 1.4. The shape of the human tibial surface is a trapezoid with an anterior-posterior ratio of approximately 1.4, and the dimension of the tibial component may be adjusted as to reflect this ratio. This provides for a relatively high adaptation of the tibial component according to the present invention to the in vivo situation. This enables a relatively good transfer of forces.

In an embodiment of the present invention, said at least one stabilisation element and/or said fixation fin is substantially rounded at a front edge. The tibial component is placed in the tibia from the posterior side or back side. For this, a slot has to be provided in the bone. By providing a round edge at the front of the tibial component, it is relatively easy to place the tibial component into the tibia via the slot. The round edge enables a smooth placement, without damaging the bone around the slot. Furthermore, the round edge prevents bone damage at the anterior or front side of the slot. Also, it is relatively easier for the bone to attach to a round edge. Moreover, by providing a round edge, stresses on the bone and on the tibial component are minimised.

The stabilisation element of the tibial component according to the present invention may be made substantially of Cobalt Chromium, wherein the outer surface of said at least one stabilisation element is provided with a Titanium Plasma Spray coating. The tibial component may be made of cast CrCoMo alloy. During the casting process bondings between carbon and metals are formed. These so-called carbides add to the hardness of the material and the stiffness of the implant. A drawback to this is that the carbides themselves are much harder than CoCrMo. Where these carbides protrude from the surface of the implant they may cause harm to the opposite articulating surfaces by scratching them. It is even possible for carbides to break out and cause third-body wear. These detrimental effects can result in an accelerated wear of the polyethylene and even cause aseptic loosening. Fortunately, a number of techniques is available today to provide a smoother articulating surface. Heat treatment and high polishing of the surfaces can help reduce the amount of wear but they still cannot completely eliminate carbides from the articulating surface. To overcome this last hurdle, the tibial component may be coated with a Titanium Nitride ceramic. The ultra-hard Titanium Nitride layer forms a supremely smooth surface and covers any protruding carbides, preventing scratching of the opposite polyethylene articulating surfaces. All in all, this enhances the wear characteristics of both the tibial component and the tibia. Also, this reduces metal ion release from implant components in case of proven metal hypersensitivity.

In an embodiment of the present invention, at least a part of said outer surface is further provided with a Calcium Phosphate coating. This Calcium Phosphate coating is also known as Bonit. The Bonit coating provides the optimal environment for osseointegration. Osseointegration is the direct structural and functional connection between living bone and the surface of a load-bearing artificial implant. The Bonit coating allows the bone to connect to the tibial component and allows for a cementless fixation of the tibial component in the tibia.

In an embodiment of the present invention, said at least one stabilisation element is provided at an upper end of said fixation fin. By providing the stabilisation element at an upper end of the fixation fin, the form of the tibial component is relatively continuous. This relatively simplified form of the tibial component allows for a relatively easy construction of the slot that is needed to place the tibial component.

In an embodiment of the present invention, two stabilisation elements are provided. By providing two stabilisation elements, it is easier to balance the stabilisation of the tibial component.

In an embodiment, the two stabilisation elements extend from the fin in substantially opposing directions. It is, for instance, possible to provide a lateral and a medial stabilisation element. This improves the stabilisation of the tibial component. The lateral and medial stabilisation elements may be placed at the same angle relative to the fixation fin. It is however also possible to place the lateral stabilisation element at a different angle relative to the fixation fin than the medial stabilisation element. By doing this, it is possible to specifically adjust the stabilisation of the tibial component. In a preferred embodiment, the two stabilisation elements are placed on the fixation fin with the same angle, and the stabilisation elements extend in opposite directions.

In an embodiment, a lower surface of the base comprises an articulating surface, wherein at least a part of said articulating surface is provided with a Titanium Nitride coating.

According to another aspect of the current invention, the invention provides an ankle prosthesis comprising a tibial component as herein defined. An ankle prosthesis according to the present invention provides a relatively high stabilisation and relatively low bone abrasion. The ankle prosthesis according to the invention may be used for post traumatic arthrosis, primary arthritic and rheumatoid ankle, instability of the athritic ankle and revision of a primary ankle prosthesis.

An embodiment of the present invention will now be explained in more detail in relation to the accompanying figures, in which:
Fig. 1 an ankle prosthesis according to the state of the art;
Fig. 2a and 2b a tibial component for an ankle prosthesis according to the present invention;
Fig. 3a and 3b an ankle prosthesis according to the state of the art and a replacement tibial component according to the present invention.

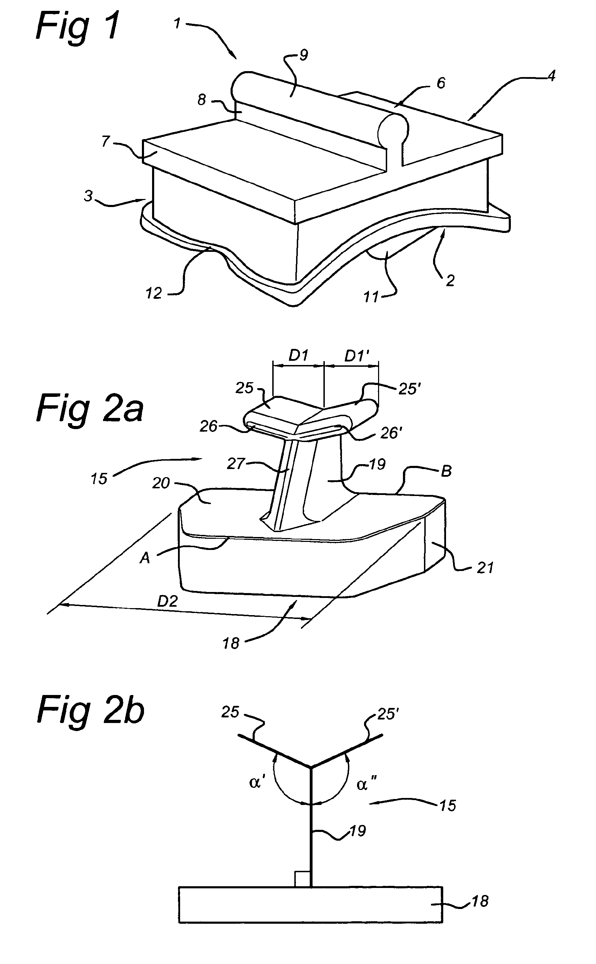

Fig. 1 shows a perspective view of an ankle prosthesis 1 according to the state of the art. The ankle prosthesis 1 shown is a so called B-P Mobile Bearing Total Ankle System, developed by Buechel and Pappas. The ankle prosthesis 1 has a talar component 2 and a tibial component 4. In between the talar component 2 and the tibial component 4, a bearing 3 is placed. The talar component has a saddle shaped surface 12. At the bottom of the talar component 2, a bone fixation portion 11 is provided. The tibial component 4 has a base 7 and a bone fixation portion 6, consisting of a rectangular block 8 and a cylindrical element 9.

Fig. 2a shows a perspective view of a tibial component 15 for an ankle prosthesis according to an embodiment of the present invention. The tibial component 15 has a base 18 with a side 21 and a top surface 20. The base has a trapezoidal shape, wherein the backside of the base has a larger width than the front side of the base. The ratio between the back side (anterior side) and the front side (posterior side) may be about 1.0 to 2.0. In the embodiment shown, the anterior-posterior ratio is equal to about 1.4. A fixation fin 19 is placed on the top surface 20 of the base 18. On the fixation fin 19, two stabilisation elements 25, 25' are provided. The two stabilisation elements 25, 25' extend in substantially opposing directions. It is possible, however, to use any number of stabilisation elements, extending in any direction suitable. In the shown embodiment, the stabilisation elements 25, 25' are provided on the upper end of the fixation fin 19. It is possible however, to place a stabilisation element 25, 25' on any part of the fixation fin 19 without departing from the invention. The stabilisation elements 25, 25' extend away from the fixation fin 19, in a substantially sideways direction. The stabilisation elements 25, 25' may be made of Cobalt Chromium. The outer surface of the stabilisation elements may be provided with a Titanium Plasma Spray coating. The outer surface may further be provided with a Calcium Phosphate coating. The front edge 27 of the fixation fin is rounded, as are the front edges 26, 26' of the stabilisation elements 25, 25'. The base 18 is substantially trapezoidal. The ratio between the back side B of the base 18 and the front side A of the base is equal to 1.4.

The stabilisation elements 25,25' extend from the fixation fin 19 with distances D1, D1'. The distance is measured from the centre line of the fixation fin 19. The distances D1, D2, are in the shown embodiment equal to 10% of the width D2 of the base 18. It is possible however, to provide the stabilisation elements 25,25' with a greater relative width D1,D2. It is also possible that a first stabilisation element 25 has a specific width D1 that is different to the width D2 of a second stabilisation element 25'.

In Fig. 2b, a schematic front view of the tibial component 15 is shown. Equal elements compared to Fig. 2a are numbered the same. The fixation fin 19 extends at a right angle from the base. At the top of the fixation fin 19, the two stabilisation elements extend sideways from the fixation fin 19. The stabilisation elements 25,25'extend at an oblique angle from the fixation fin. In the shown embodiment, the angle α, α' between the respective stabilisation elements 25, 25' with respect to the centre line of the fixation element is equal to 110°. In a preferred embodiment, this angle is in between 95° and 155°.

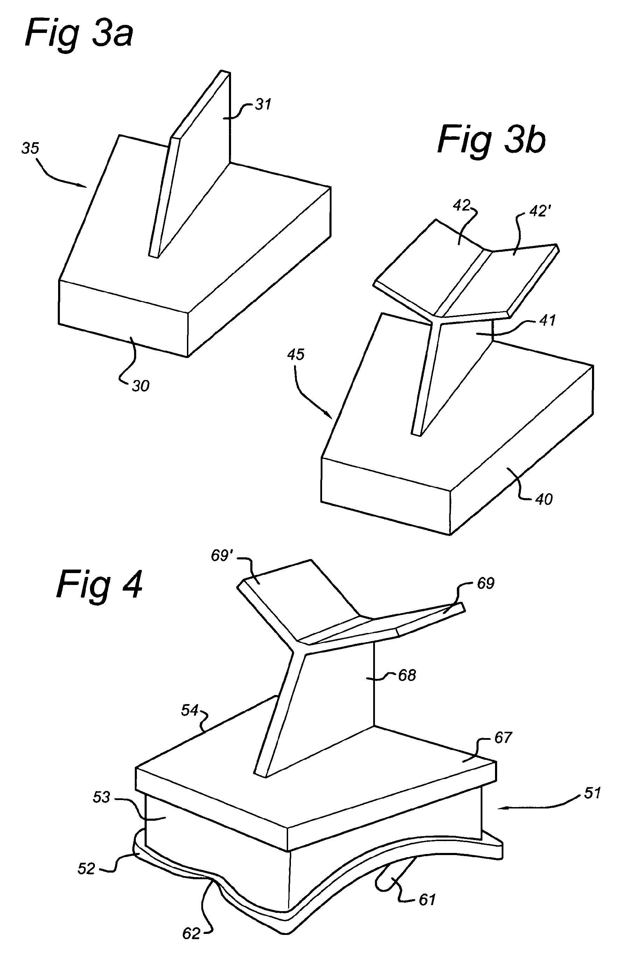

As shown in Fig. 3a and 3b, the tibial component 45 according to the present invention, and shown in Fig. 3b may be used to replace an existing tibial component 35 as shown in Fig. 3a. The tibial component according to an embodiment of the present invention has a base 40, a fixation fin 41 and two stabilisation elements 42, 42'. The tibial component 35 to be replaced has a base 30 and a fixation fin 31, and may lack the stabilisation elements 42, 42' of the tibial component according to the present invention.

With the tibial component according to the present invention, it is possible to replace an existing tibial component. This is especially useful when the cohesion between the existing tibial component and the bone is deteriorated. In this case, a similar tibial component may not be sufficient for providing enough stability for the newly placed prosthesis. The stabilisation elements on the tibial component according to the present invention provide for a relatively high stabilisation for the replacement tibial component. This allows the bone to regrow around the damaged part. Moreover, only minimal bone resection is needed when replacing a worn tibial component.

Fig. 4 shows a persepective view of an ankle prosthesis according to the present invention, including a tibial component as described herein. The ankle prosthesis 51 has a talar component 52 and a tibial component 54. In between the talar component 52 and the tibial component 54, a bearing 53 is placed. The talar component has a saddle shaped surface 62. At the bottom of the talar component 2, a bone fixation portion 61 is provided. The bone fixation portion comprises two cylindrical elements (only one being shown in Fig. 4), extending obliquely from the bottom of the talar component. The tibial component 64 has a base 67 and a fin 68. On the fin, a wing is provided that functions as stabilisation elements 69,69'.

It will be understood that various details of the presently disclosed subject matter may be changed without departing from the scope of the subject matter. Furthermore, the foregoing description is for the purpose of illustration only, and not for the purpose of limitation.

## Claims

1. Tibial component (15; 54) for use in an ankle prosthesis (51), wherein said tibial component (15) comprises:
- a base (18; 67) having an upper surface (20);
- a fixation fin (19; 68) protruding generally upwardly from the upper surface (20) of the base (18) and extending in an anterior-posterior direction along the base (18); and
- at least one stabilisation element (25; 69) provided on the fixation fin (19) for stabilisation of the tibial component (15) when placed in the tibia, said at least one stabilisation element (25) extending sideways away from the fixation fin (19) and at an oblique angle (α') relative to the upward direction of the fixation fin.

2. Tibial component (15) according to claim 1, wherein said angle (α') is between 95° and 155°.

3. Tibial component (15) according to claim 1, wherein the tibial component (15) can be used as a replacement tibial component for replacing a worn out tibial component in an earlier placed ankle prosthesis.

4. Tibial component (15) according to claim 1, wherein said at least one stabilisation element (25) extends a distance of at least 10% of a width of said base (18).

5. Tibial component (15) according to any one of the preceding claims, wherein said base is substantially trapezoidal having an anterior-posterior ratio of about 1.0 to 2.0, preferably of about 1.4.

6. Tibial component (15) according to any one of the preceding claims, wherein said at least one stabilisation element (25) and/or said fixation fin (19) is substantially rounded at a front edge.

7. Tibial component (15) according to any one of the preceding claims, wherein said at least one stabilisation element (25) is made substantially of Cobalt Chromium, wherein the outer surface of said at least one stabilisation element (25) is provided with a Titanium Plasma Spray coating.

8. Tibial component (15) according to claim 7, wherein at least a part of said outer surface is further provided with a Calcium Phosphate coating.

9. Tibial component (15) according to any one of the preceding claims, wherein said at least one stabilisation element (25) is provided at an upper end of said fixation fin (19).

10. Tibial component (15) according to any one of the preceding claims, wherein two stabilisation elements (25, 25') are provided, wherein the two stabilisation elements (25, 25') preferably extend from the fin (19) in substantially opposing directions.

11. Tibial component (15) according to any one of the preceding claims, wherein a lower surface of the base comprises an articulating surface, wherein at least a part of said articulating surface is provided with a Titanium Nitride coating.

12. Tibial component (15) according to claim 1, wherein the fixation fin (19) is provided with a wing as stabilisation elements for stabilisation of the tibial component.

13. Ankle prosthesis (51) comprising a tibial component (54) according to any one of the preceding claims, a talar component (52), and a bearing (53) component for placement between said tibial and said talar component.

## Patentansprüche

1. Tibiakomponente (15; 54) zur Verwendung in einer Fußgelenkprothese (51), wobei die Tibiakomponente (15) Folgendes umfasst:
- eine Basis (18; 67) mit einer oberen Fläche (20);
- eine Fixierungsrippe (19; 68), die von der oberen Fläche (20) der Basis (18) im Allgemeinen nach oben ragt und entlang der Basis (18) in anterior-posterior-Richtung verläuft; und
- mindestens ein Stabilisierungselement (25; 69), das an der Fixierungsrippe (19) bereit gestellt ist, um die Tibiakomponente (15) beim Einsetzen in die Tibia zu stabilisieren, wobei das mindestens eine Stabilisierungselement (25) seitlich von der Fixierungsrippe (19) weg und in einem schrägen Winkel (α') im Verhältnis zur nach oben gerichteten Fixierungsrippe verläuft.

2. Tibiakomponente (15) nach Anspruch 1, wobei der Winkel (α') zwischen 95° und 155° beträgt.

3. Tibiakomponente (15) nach Anspruch 1, wobei die Tibiakomponente (15) als Tibiakomponentenersatz verwendet werden kann, um eine abgenutzte Tibiakomponente in einer zu einem früheren Zeitpunkt eingesetzten Fußgelenkprothese zu ersetzen.

4. Tibiakomponente (15) nach Anspruch 1, wobei das mindestens eine Stabilisierungselement (25) über eine Strecke von mindestens 10 % einer Breite der Basis (18) verläuft.

5. Tibiakomponente (15) nach einem der vorhergehenden Ansprüche, wobei die Basis im Wesentlichen trapezförmig ist und ein anterior-posterior-Verhältnis von ungefähr 1,0 bis 2,0, vorzugsweise ungefähr 1,4, aufweist.

6. Tibiakomponente (15) nach einem der vorhergehenden Ansprüche, wobei das mindestens eine Stabilisierungselement (25) und/oder die Fixierungsrippe (19) an einer vorderen Kante im Wesentlichen gerundet sind.

7. Tibiakomponente (15) nach einem der vorhergehenden Ansprüche, wobei das mindestens eine Stabilisierungselement (25) im Wesentlichen aus Cobalt-Chrom gefertigt ist, wobei die Außenfläche des mindestens einen Stablilisierungselements (25) mit einer Titanplasmaspray-Beschichtung versehen ist.

8. Tibiakomponente (15) nach Anspruch 7, wobei mindestens ein Teil der Außenfläche ferner mit einer Calciumposphat-Beschichtung versehen ist.

9. Tibiakomponente (15) nach einem der vorhergehenden Ansprüche, wobei das mindestens eine Stabilisierungselement (25) an einem oberen Ende der Fixierungsrippe (19) bereit gestellt ist.

10. Tibiakomponente (15) nach einem der vorhergehenden Ansprüche, wobei zwei Stabilisierungselemente (25, 25') bereitgestellt sind, wobei die zwei Stabilisierungselemente (25, 25') vorzugsweise von der Rippe (19) ausgehend in im Wesentlichen entgegengesetzten Richtungen verlaufen.

11. Tibiakomponente (15) nach einem der vorhergehenden Ansprüche, wobei eine untere Fläche der Basis eine Gelenkfläche aufweist, wobei mindestens ein Teil der Gelenkfläche mit einer Titannitrid-Beschichtung versehen ist.

12. Tibiakomponente (15) nach Anspruch 1, wobei die Fixierungsrippe (19) einen Flügel als Stabilisierungselemente zum Stabilisieren der Tibiakomponente aufweist.

13. Fußgelenkprothese (51), die eine Tibiakomponente (54) nach einem der vorhergehenden Ansprüche, eine Taluskomponente (52) und eine tragende (53) Komponente zum Einsetzen zwischen die Tibia- und die Taluskomponente umfasst.

## Revendications

1. Composant tibial (15 ; 54) à utiliser dans une prothèse de cheville (51), dans lequel ledit composant tibial (15) comprend :
- une base (18 ; 67) ayant une surface supérieure (20) ;
- une ailette de fixation (19 ; 68) faisant saillie généralement vers le haut depuis la surface supérieure (20) de la base (18) et s'étendant dans une direction antérieure-postérieure le long de la base (18) ; et
- au moins un élément de stabilisation (25 ; 69) agencé sur l'ailette de fixation (19) pour stabilisation du composant tibial (15) lorsqu'il est placé sur le tibia, ledit au moins un élément de stabilisation (25) s'étendant latéralement en éloignement de l'ailette de fixation (19) et à un angle oblique (α') par rapport à la direction vers le haut de l'ailette de fixation.

2. Composant tibial (15) selon la revendication 1, dans lequel ledit angle (α') mesure entre 95° et 155°.

3. Composant tibial (15) selon la revendication 1, dans lequel le composant tibial (15) peut être utilisé comme composant tibial de substitution pour remplacer un composant tibial usé dans une prothèse de cheville placée antérieurement.

4. Composant tibial (15) selon la revendication 1, dans lequel ledit au moins un élément de stabilisation (25) s'étend sur une distance d'au moins 10 % d'une largeur de ladite base (18).

5. Composant tibial (15) selon l'une quelconque des revendications précédentes, dans lequel ladite base est sensiblement trapézoïdale ayant un rapport antérieur-postérieur d'environ 1,0 à 2,0, de préférence d'environ 1,4.

6. Composant tibial (15) selon l'une quelconque des revendications précédentes, dans lequel ledit au moins un élément de stabilisation (25) et/ou ladite ailette de fixation (19) sont sensiblement arrondis au niveau d'un bord avant.

7. Composant tibial (15) selon l'une quelconque des revendications précédentes, dans lequel ledit au moins un élément de stabilisation (25) est constitué sensiblement de cobalt-chrome, dans lequel la surface externe dudit au moins un élément de stabilisation (25) est dotée d'un revêtement de pulvérisation par plasma de titane.

8. Composant tibial (15) selon la revendication 7, dans lequel au moins une partie de ladite surface externe est en outre dotée d'un revêtement de phosphate de calcium.

9. Composant tibial (15) selon l'une quelconque des revendications précédentes, dans lequel ledit au moins un élément de stabilisation (25) est agencé au niveau d'une extrémité supérieure de ladite ailette de fixation (19).

10. Composant tibial (15) selon l'une quelconque des revendications précédentes, dans lequel deux éléments de stabilisation (25, 25') sont fournis, dans lequel les deux éléments de stabilisation (25, 25') s'étendent de préférence à partir de l'ailette (19) dans des directions sensiblement opposées.

11. Composant tibial (15) selon l'une quelconque des revendications précédentes, dans lequel une surface inférieure de la base comprend une surface d'articulation, dans lequel au moins une partie de ladite surface d'articulation est dotée d'un revêtement de nitrure de titane.

12. Composant tibial (15) selon la revendication 1, dans lequel l'ailette de fixation (19) est dotée d'une aile comme éléments de stabilisation pour stabilisation du composant tibial.

13. Prothèse de cheville (51) comprenant un composant tibial (54) selon l'une quelconque des revendications précédentes, un composant astragalien (52), et un composant porteur (53) pour un placement entre lesdits composants tibial et astragalien.
